# EUROPEAN PATENT APPLICATION

(11) **EP 3 660 502 A1**
(43) Date of publication of application: **03.06.2020**
(21) Application number: 18460070.8
(22) Date of filing: 29.11.2018
(51) Int. Cl.: G01N 30/00, G01N 33/49

(54) **A METHOD FOR DETERMINATION OF 5-FLUOROURACIL IN SERUM AND A KIT FOR THEREOF**

(71) Applicant: ALAB Laboratoria Sp. z o.o., 00-739 Warszawa (PL)
(72) Inventor: Budzynska, Beata, 05-820 Piastów (PL); Celejewska, Agnieszka, 20-538 Lublin (PL); Dobosz, Malgorzata, 01-237 Warszawa (PL); Grzybowska, Marta, 01-471 Warszawa (PL); Kobiela-Mednis, Agnieszka, 03-938 Warszawa (PL); Starczewski, Konrad, 01-493 Warszawa (PL); Strumnik-Filipek, Anna, 02-979 Warszawa (PL); Wardak, Sebastian, 01-858 Warszawa (PL); Zagraba, Monika, 21-010 ??czna (PL); Zakrzewski, Marcin, 03-042 Warszawa (PL); Kosno, Katarzyna, 01-912 Warszawa (PL); Kromplewska, Anna, 85-436 Bydgoszcz (PL); Paprotny, Lukasz, 03-994 Warszawa (PL); Pietrala, Malwina, 05-850 Ozarów Mazowiecki (PL); Placzek, Andrzelika, 08-210 Kolonia Ostromeczyn (PL); Wianowska, Dorota, 20-508 Lublin (PL)

(57) **Abstract**

The subject of the invention is a way of 5-FU quantification in blood serum with an internal standard using HPLC with a diode detector, including preparation stage of standard solutions, sample preparation and quantification performance, characterised by the fact that sample is prepared with zirconium sorbing agent and quantification takes place on the HYDRO-RP column at 265 nm wave length.

The subject of the invention is also the performance set.

## Description

The subject of the invention is a way of 5-FU quantifications in blood serum and a set for performing this. The invention pertains to laboratory diagnostics area.

Currently mainly immmunochemical methods meaning detection of examined substances based on an antigen-antibody reaction are taken advantage of in toxicology. Immmunochemical methods require a chemical structure showing affinity of a specific antibody. In diagnostics this creates a huge problem, since, until nowadays, there's no full range of antibodies and, quite often, their affinity level and selectivity are insufficient. Interferences in laboratory diagnostics can be defined as the impact of a substance present in the examined sample involving a change of the correct value of a given parameter, usually expressed as concentration or activity. The most important include: - matrix effects (the lower the sample volume in relation to reaction mix, the lower likelihood of an interference - heterogeneity of the sampled analyte quantification - cross reactions - an antibody bonding with a substance different from the analyte - factors influencing the change of concentration (e.g. stress, infections, allergies, contagions, diet). In the case of immmunochemical methods the first three are of special significance and thus the method rules out high testing accuracy of many substances. A drawback of quick tests is the diversity of possible interferences which can be a cause of getting so-called false positive or false negative results.

Cytostatic drugs are a group of natural and synthetic substances used in cancer chemotherapy, having toxic effects on cancer cells characterised by quick cell divisions. However, the drugs also damage other quick-dividing yet healthy cells (bone marrow, mucous membranes, hair follicle cells), thus adverse effects, such as anaemia, nausea and vomiting, alopecia) are quite common. Low therapeutic index combined with quantification drawbacks of immmunochemical methods (including low sensitivity - antibody release threshold, low selectivity - possibility of interactions, high readout error) cause significant difficulties in effective therapy and sometimes even make proper therapy impossible. The attending physician, despite exercising the planned therapy, can do more damage to a patient than failing to apply a pharmacotherapy. A test result may show a level being right for the patient, but also showing a toxic level within the error. Considering this it's necessary to develop accurate analytical methods and new calibrating sets to work out a method providing correct quantification results in order to monitor cytostatic drugs concentration in the course of deployed therapies.

There's also a demand for developing ready analytical sets that will make it possible for a physician to monitor drugs concentration during the therapy. It's of special significance when considering a high therapeutic spectrum drugs, non-linear pharmacokinetics, lack of a direct interrelation between the applied dose and therapeutic effect and a significant diversity of intra and inter-individual course of pharmacokinetics. Owing to this a physician will be able to observe an interrelation between constitutional drug content in the organism and the therapeutic effect, with no worries about a false result caused by presence of a series of interferences. The therapeutic effects of so-called critical dose drugs is practically impossible to achieve during a standard therapy, thus an individualized therapy is often used and, as a result of it, it's necessary to adjust a dose to a patient's individual needs.

Thus currently the main obstacle in obtaining clinical efficiency are toxic effects in fine and healthy tissues, since a decreased drug dose, not being accurately adjusted to a patient's parameters, may cause an adverse effect. Thereby application of, for instance, cancer drugs may be fundamentally beneficial when their dosage is precisely adjusted to individual needs of the patient and methods enabling the drug concentration in patient's blood are accurate, quick, relatively cheap and possible in hospital-based diagnostic conditions.

5-fluorouracil (5-FU) is an organic chemical compound from the group of pyrimidine bases; a fluoric derivative of uracil. It's a cytostatic belonging to the group of pyrimidine antimetabolites and it's used as a phase-specific anticancer drug (phase S).

5-FU is one of the most often used chemotherapeutic agents during bowel, oesophageal, rectal, breast, skin, head and neck-area cancer therapy. The main enzyme metabolising the drug is dihydropyrimidine dehydrogenase (DPD), encoded by the DPYD gene, responsible for catabolising approx. 80% if the applied 5-FU. In patients with DPD deficiency there's a huge risk that a severe, sometimes lethal, toxicity to this chemotherapeutic may develop. Non-linear pharmacokinetics of 5-FU and a very low therapeutic index considerably hinder prognostication of an organism reactions after a standard drug dose application. Thus it's crucial to individually adjust the optimum dose, so that to get the best possible therapeutic effect with minimum adverse effects.

The generally adopted dose adjustment method, based on the BSA index, does not make it possible to determine such drug concentration that would guarantee the highest treatment efficiency guaranteeing its safety at the same time.

There are many works and researches showing the necessity of dihydropyrimidine dehydrogenase activity quantification prior to 5-FU administration, which should make it possible to adjust a dose enabling the desired and optimum drug concentration in the patient's blood without over-toxic exposure. Currently, despite huge demand for 5-FU quantification in a biological material of human origin, diagnostic labs are not able to perform such analyses as a standard, as this is related to lack of ready reagents sets and the fact that a very high lipophobicity of this compound causes many problems pertaining to HPLC, and even LC-MS/MS methodology.

Developing a procedure including DPD activity quantification, determining the 5-FU dose and monitoring concentration of this chemotherapeutic during the therapeutic course may become a basis to work out new therapeutic standards in oncology and thus significantly influence the quality and span of patients' life (Angelika Szcz 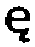 śniak at al, Postepy Hig. Med. Dosw. (online), 2018; 72: 81-88) Quantification of cytotoxic agents using the LC-MS/MS method is known. The publication by Barbara Büchel at al, Biomedical Chromatography, approved for publishing on July 14th 2011, described the LC-MS/MS method for simultaneous analysis of uracil, including 5-FU. The sample was prepared by adding 200 µl of a blood serum sample with 10 µM serum concentration for 5-FU quantification. The serum proteins were precipitated with ammonium sulphate solution and the analyte was extracted. After vortexing and mixing, the sample was centrifuged and the supernatant was dried in polypropylene. After re-extraction and drying, the sample was diluted in 0.1% solution of formic acid and exposed to ultrasounds. The extract was centrifuged and the clean supernatant was placed in vials to undergo LC-MS analysis using apparatus by Agilent Technologies, with Arlantis dC18 column (2.1x150mm i.d.; particles size 3µm) connected to Arlantis dC18 pre-column (2.1x10mm i.d.; particles size 3µm) by Waters. The mobile stage contained 1.0 mM of ammonium acetate in water, 0.5 mM of formic acid and 3.3% of methanol, and 0.2 ml/min flow was used. 10 ml of sample at 5 C. temperature was injected for 10 mins. MS analysis was performed on 4000 QTRAP LC/MS/MS system from AB SCIEX from MRM. Special settings not referred to herein were used. The quantification was performed using an internal standard method. The analysis was performed on Analyst software, v. 1.5 (AB Sciex). Selection of analytical parameters caused the analyte was correctly ionized, using forward bias. The method proved right for 5-FU metabolite levels quantification at 0,1-75 µm) level in human blood serum. According to the authors, the method can be used in standard clinical applications for 5-FU monitoring. The described way is however possible only when a mass detector is used, what makes performance of standard analyses in hospital-based conditions difficult and also increases the costs of such analysis.

Methods of 5-FU and 5,6-Dihydro-5-fluorouracil analysis using the HPLC method, with diode detection, are also known for the purpose of cell activity quantification of dihydropyrimidine dehydrogenase and pharmacokinetic profile - Antonello Di Paolo i in. Ther. Drug. Monit. tom [volume] 27, No. 3, July 2005. The author describes a procedure when 5-FU and 5-FDHU are extracted from a biological material by adding sodium acetate, sodium sulphate and diethyl ether/propanol. The dried samples were diluted in the mobile phase (KH₂PO₄ 35 mmol/l, pH 4.0), isocratically washed on the Hypersil C18 column (25 cm x 4.6 mm, 10 µm) taking advantage of diode detection at 215 and 360 nm wave length. The peaks' retention occurred about the 13th minute. The method proved positive recovery, linearity, detection limit parameters and the quantification was 3.2 and 16 ng/ml adequately for 5-FDHU and 5-FU. A drawback of this method is, for instance, the need of using non-stable buffers, as constituents of the mobile phase, being quite often a reason for HPLC unit impurity and growth of algae and bacterial flora.

The invention, pursuant to this notification, is meant to meet the drawbacks of 5 quantification, as known in the current state of technique.

The invention pertains to a way of 5-FU quantification in blood serum with an internal standard, using HPLC with a diode detector, including the sample solution preparation stage, sample preparation and performing the quantification, and is characterised by the fact that a sample is prepared with zirconium sorbing agent and the quantification is done on a HYDRO-RP column at 265 nm wave length.

Favourably the 5-FU quantification includes the following stages:
a. preparing standard solutions, i.e.:
   - preparing solutions of basic standards - basic solution of 5-FU; Cₚ 5-FU of 9.6 mg/ml concentration in water and basic solutions of 5-bromouracil, C_{p IS} of 4.5 mg/ml concentration in 50 % ACN.
   - preparing working solution of 5-fluorouracil - working solution I 5-fluorouracil; Cₚ 5-FU in water of 10 ug/ml concentration (Cr 5-FU_I), working solution II 5-fluorouracil; Cₚ 5-FU in water of 1.0 µg/ml concentration (Cr 5-FU_II), working solution III 5-fluorouracil; Cₚ 5-FU in water of 0.1 µg/ml concentration (Cr 5-FU_III)
   - preparing working solution of an internal standard (5-bromouracil) - working solution of IS; C_{r IS} in 20% MeOH of 0.058 µg/ml concentration (C_{r IS}).
b. preparing a sample, i.e.:
   - sampling 200 µl blood serum and 6.9 µl of 5-FU internal standard solution of 0.058 µg/ml concentration in 20% MeOH (C_{r IS}),
   - adding 300 mg of ammonia sulphate, 100 mg zirconium sorbing agent - Z-Sep and 1 ml of 15 % solution of 2-propanol in ethyl acetate,
   - stirring such prepared mix for 10 minutes
   - centrifuging at 20 000 x g for 5 minutes
   - evaporating 600 ul of the extract in nitrogen stream
   - reconstituting such obtained dry residue in 200 ul 20 % of methyl alcohol solution;
c. performing an essay on HPLC, where:
   - mobile phase: MeOH/H₂O of 5/95 % vol/vol content adequately
   - dosed volume: 10 µl
   - measurement temperature: 25 °C
   - mobile phase flow speed: 1.0 ml/min

Favourably the way of monitoring 5-FU concentration in blood serum by means of a series of quantifications, following the aforementioned procedure.

The invention also pertains to a performance set, according to the invention including:
- Synergi Hydro-RP column, Phenomenex, dimensions 150 x 4.6 mm, 4 µm grain, pores diameter 80A,
- pre-column, Phenomenex, AQ C18 4x3.0 mm,
- 100 vials with an insert,
- 100 eppendorfs
- mobile phase: 950 ml water to LC and 50 ml MeOH to LC
- 20% solution of MeOH - 20ml
- 15% solution of isopropanol in ethyl acetate - 100ml
- ammonium sulphate - 300g
- sorbing agent - Z-Sep Supelco - 10g
- internal standard of 5-bromouracil
- a calibrating device: 5-FU
- analysis performing instruction.

The HPLC methodology using the developed way and chromatographic sets enables a quick modification of a drug dose, depending on the observed diversity in an active substance absorption from the form of a drug, the course of metabolism, or presence of interactions between the administered drugs, e.g. during coadministration. Undoubtedly, as far as medicine is concerned, determining safe therapeutic concentration ranges for some drugs will make it possible to minimize, e.g. episodes of acute organ transplant rejections or occurrence of other undesired phenomena in a human organism undergoing a therapy.

In such case the solution will be measurable in a form of decreased costs of therapy, improved quality of patients' life and knowledge of extra parameters showing the drug's ups and downs in the organism. Owing to a relatively low price of HPLC apparatus and its relatively easy operation, the analysis can be performed even by personnel not qualified in the field, in any clinical unit taking advantage of this drug therapy.

Attention needs to be paid to hazards appearing during interpretation of results for the concentration values quantified by means of traditional methods (such as low concentration values and drugs exposure in organism). The method and the set can be used for a standard therapy monitored by the drug level in blood. An effect of using this invention is the opportunity of pharmacologic treatment depending, first and foremost, on keeping a therapeutic drug concentration level in the organism, a level which varies in particular patients and which may depend on biological factors, as well as environmental ones, e.g. diet or coadministration. This is particularly important in the case of taking pharmaceuticals of a narrow range between a therapeutic and toxic dose (i.e. low therapeutic index), as is the case in cancer therapy.

Determining safe therapeutic concentration ranges for cancer drugs will minimize occurrence of unsafe adverse effects episodes in a human organism.

Owing to the method referred to in the invention and developing ready sets and standards for chemical substances, one eliminates the need of administering a huge dose of harmful reagents, as it has been so far in the case of traditional methods, such as immmunochemical ones, which force application of considerable amounts of chemical, highly toxic substances, in particular sodium azide or mercury compounds acting as preservatives of analytical reagents. In this case, following the invention, preservatives are the phase constituents, e.g. methyl alcohol, isopropanol or acetonitrile. Deploying selected sets for specific substances decreases the use of harmful substances (e.g. phosphoric acid, fluoric acid, sodium azide, heavy metals compounds) even up to 100% in comparison to immunologic methods, or, for instance, still showing great interest, thin-layer chromatography (TLC). The aforementioned sodium azide is a highly toxic and mutagenic substance, easily absorbable through the skin. Both compounds require use of protective gloves. It's easily soluble in water, odourless and colourless.

Taken advantage of based on the invention, zirconium sorbing agent undoubtedly distinguishes the methodology from previous works, on the basis of immmunochemical and chromatographic ways. This enables very effective purifying of such complex and complicated matrix as human blood serum, simultaneously adding to a very significant increase of selectivity, since it adsorbs low-particle compounds of very high hydrophilicity and of a very polar particles, similarly as the quantified 5-FU, what prevents occurrence of potential cross reactions which, in this case, mean co-presence of signals in the form of chromatographic peaks in the same retention time. It was surprising to determine that 5-FU is not adsorbed by zirconium sorbing agent and is not "lost" as a result of sample preparation, and thus is characterised with a perfect recovery result. This is important, since 5-FU, being a chemotherapeutic, is characterised with a very narrow therapeutic index, i.e. its therapeutic concentration is very close to toxic one. Due to the fact the way described in the invention is highly selective (as proved in experiments based on the analysis of test results obtained for such parameters as recovery, method sensitivity, accuracy, reconstruction, repeatability, and many more described below) and significantly increases safety of the applied chemotherapy, surely adding to increased percentage of "curability" of individuals suffering from a cancer.

Summing up, the developed procedure and its performance set, thanks to unique materials taken advantage of during the sample preparation stage (zirconium sorbing agent) and analysis (HYDRO column), makes it possible to:
- perform the quantification with HPLC apparatus with an UV or DAD detector, which are relatively cheap and simple to use, thus increasing application possibilities of this method;
- increase accuracy and repeatability, thanks to using the adequate internal standard;
- obtain sensitivity comparable to expensive, as far as purchase and operation are concerned, LC-MS devices, which additionally require highly-qualified staff to operate;
- increase selectivity of liquid-liquid extraction, which is simple, easy to use and additionally does hot require specialist equipment;
- limit presence of qualified staff during analyses
- minimize the quantity of unsafe and expensive organic solvents and, as a result, it generates little waste and radically decreases the costs of its management;
- shorten the result time of an analytical procedure and
- shorten the time of analysis, both using the invention-based set, but also thanks to the retention time for 5-FU, achieved through the invention and decreased in comparison to currently known solutions
- considering the aforementioned, the number of quantifications necessary to perform is increased and their high accuracy and precision are guaranteed.

### Definitions:

5-FU - 5-fluorouracil
5-BU - 5-bromouracil
IS - Eng. internal standard
LLOQ - Eng. Lower Limit of Quantification
PPI - Eng. Peak Purity Index
ULOQ - Eng. Upper Limit of Quantification
Bias - accuracy (inconsistency level for results obtained using a given method with the value assumed as correct) reflecting a systematic error, expressed in % ?
CV - Critical Value, critical value of reconstruction, repeated result, and non-precision of reconstruction accuracy for a measurement result, expressed in %
Cₚ - basic solution concentration
C_{r IS} - working solution concentration for an internal standard
SST - Eng. System Suitability Test

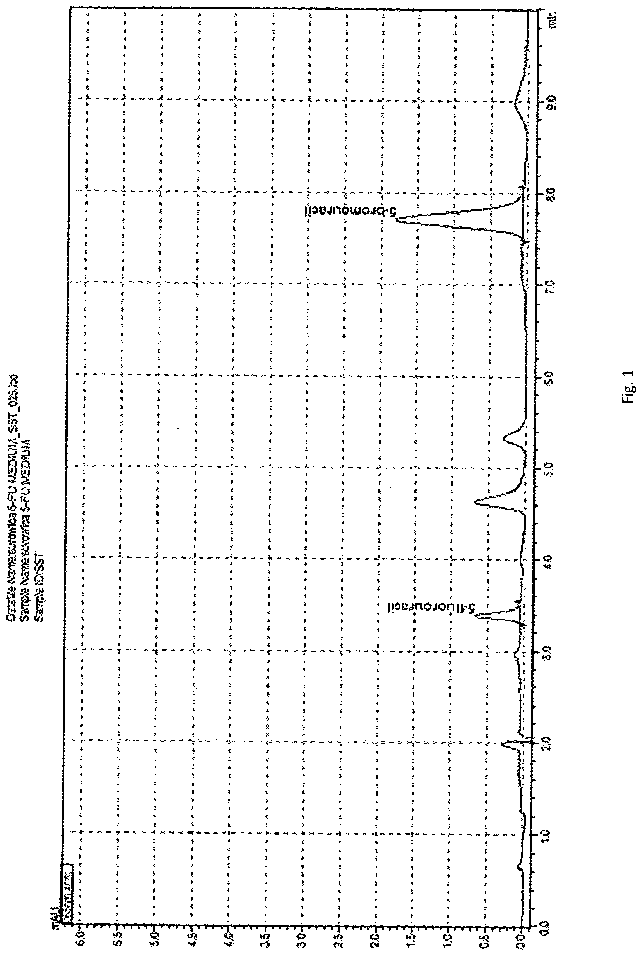

A sample chromatogram performed in the bay based on the invention is presented in Fig. 1

### Examples

Invention-based quantification was performed.

During the tests the following reagents, materials and basic laboratory equipment were used:
- Blood serum free from 5-FU and internal standard (5-BU);
- Acetonitrile (ACN), HPLC purity, series No. 10901330 729, BB date 31/07/2020;
- methanol, HPLC purity, series No. I0894707 725, BB date 31/05/2020;
- ammonium sulphate;
- 2-propanol (isopropanol);
- Z-Sep Supelco sorbing agent;
- deionized water, Type I (18,2 M 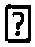 ) from Milli-Q system by Millipore;
- laboratory glass: 1, 2 and 5 ml capacity measurement bulbs; 2 ml vials for HPLC;
- eppendorf-type test tubes;
- analytical sales - Precisa 92 SM-202A;
- laboratory centrifuge - mySPIN 12 Thermo Scientific;
- automatic evaporation/concentration system - Xcel Vap
- vortex shaker - Thermo Scientific;
- automatic pipette 2- 20 µl Eppendorf Research
- automatic pipette 10 - 100 µl Thermo Scientific
- automatic pipette100 - 1000 µl Thermo Scientific

### Preparing basic standards solutions

Basic solutions of 5-fluorouracil; Cₚ 5-FU of 9.6 mg/ml concentration in water.

Basic concentration of 5-bromouracil, C_{p IS} of 4.5 mg/ml concentration in 50 % ACN.

Solutions were stored at -18 °C.

### Preparing a working solution of 5-bromouracil

Working solution I 5-fluorouracil; Cₚ 5-FU in water of 10 ug/ml concentration (Cr 5-FU_I)
Working solution II 5-fluorouracil; Cₚ 5-FU in water of 1.0 µg/ml concentration (Cr 5-FU_II)
Working solution III 5-fluorouracil; Cₚ 5-FU in water of 0.1 µg/ml concentration (Cr 5-FU_III)

The solution was stored at -18 °C.

### Preparing an internal standard working solution (5-bromouracil)

Working solution IS; C_{r IS} in 20% MeOH of 0.058 µg/ml concentration (C_{r IS}).

The solution was stored at -18 °C.

### Preparing calibration solutions

A series of calibration working solutions were obtained from 5-fluorouracil of 0.1; 1,0 and 10.0 µg/ml concentration (Cr 5-FU_I; Cr 5-FU_II; Cr 5-FU_III) with an additive of precisely known and identical quantity of the internal standard (IS concentration in 58 ng/ml solution) and blood serum free from examined substances and the internal standard.

### Preparing control samples

Control samples were prepared on blood serum free from examined substances and the internal standard, by fortifying with solutions of 5-FU standard of 10.0 µg/ml and 1.0 µg/ml concentration (Cr 5-FU_I; Cr 5-FU_II) with an additive of a well-known and identical quantity of the internal standard (IS concentration in solution 58 ng/ml).

### Preparing the sample for chromatographic analysis

Using a pipette measure exactly 200 µl of blood serum and 6,9 µl of the internal standard solution (C_{r IS}) into an eppendorf-type test tube (in the case of calibration and control test tubes, add adequate volume of 5-FU working solution). Then add 300 mg of ammonium sulphate,100 mg of Z-Sep and 1 ml 15 % solution of 2-propanol in ethyl acetate. Gently mix the test tube content for 10 minutes, then centrifuge (20 000 x g, 5 min). Then evaporate 600 ul of the extract in nitrogen stream and reconstitute such obtained dry residue in 200 ul 20 % solution of methyl alcohol and subject it to a chromatographic analysis.

### Performing the quantification - conditions

Quantification was performed on a liquid chromatograph - NEXERA X2 (Shimadzu, Japan) equipped with: SIL-30ACXR autosampler; LC-30AD pump, DGU-20A5R gas removing unit; CTO-20AC oven; PDA SPD30A detector and data acquisition software - LabSolutions.

5-FU content is blood serum is analysed by means of a liquid chromatograph provided with a photodiode detector. Quantification performance conditions as below:

| | |
|---|---|
| **HPLC column with a pre-column** | Synergi™ Hydro-RP column, Phenomenex; dimensions: 150 x 4,6 mm, 4 µm grain, pores diameter 80 Å (s/n: H15-207736, b/n: 5375-0082) pre-column by the same company: AQ C18 4x 3.0 mm (p/n: AJ0-7511, PRD-034889) |
| **Mobile phase** | MeOH/H₂O 5/95 % v/v |
| **Flow speed** | 1.0 ml/min |
| **Column operation temperature** | 25 °C |
| **Monitored length (λ)** | 265 nm |
| **Dosed sample volume** | 10 µl |
| **SST system check-up** | In the standard solution for SST resolution between 5-FU and the internal standard peaks should be no less than 2. |
| | 5-FU and internal standard retention time is, adequately, 3.4 and 7.6 min, and the relative standard deviation for the surface fields for the next 6 dosages of the standard solution should not exceed 2%. |

The test was performed on a blood serum sample free from examined substances and the internal standard, which was enriched with exactly known quantity of 5-FU (0,4 µg/ml) and the internal standard. 5-FU and the internal standard retention time is, adequately, 3.39 and 7.67 min, the relative standard deviation of retention time values and surface area of both peaks for the next 6 dosages of the standard solution should not exceed 2 %. RS between the peaks reflecting the examined substance and the internal standard - min. 2.

### Method validation

The method, based on the invention, has been validated. Within the validation scope, the following were checked:

### A. Specificity

In order to determine the method specificity, auxiliary substances: (e.g. solvents, mobile phase, blank sample) free from the examined substance and the internal standard was prepared. Measured signal) surface area under the analyte peak and the internal standard) should only come from the quantified compound. Lack of interfering substances is determined by:
- comparison of signals received during the analyte and internal standard retention for the blank sample and an LLOQ level sample; the detector's response during analyte retention for the blank sample should constitute < 20% of the signal determined in the LLOQ sample and, during IS retention < 5% of the signal.
- following the analysis of spectral purity of absorption spectrum during elution of the analyte and the internal standard peak; expected PPI>95%.

Resolution between peaks of the matrix interfering substances, located closest to: examined substance (5-FU/5-BU) and the internal standard, and 5-FU/5-BU peaks and IS should be no less than 1.5. (Resolution values below 1.5 should also be taken into account in auxiliary data). Resolution is determined for at least five different blood serum samples including: lipemic, haemolytic and icteric serum.

Peaks of interfering substances occurring close to the analyte retention time and IS were not observed.
- signals received during the analyte retention and IS in the blank sample are:
   12.33 % for 5-FU,
   0.23 % for IS.
- spectral purity of 5-FU= 99>97% and for the internal standard 99,95 %.

An example chromatograph showing 5-FU peak and the standard peaks is shown in Fig. 1.

### B. Linearity

Linearity was assessed for a blank sample and at least 6 non-zero concentration levels. The approval criterion for calibration solutions is to obtain concentration (calculated from a calibration curve equation) not varying from the nominal concentration by more than 15%. An exception is concentration at LLOQ level and, in this case, the calculated value does not exceed 20% of nominal concentration. The interrelation is constant and reconstructible and adjusting to the straight line at the level: R² ≥ 0,995.

### C. Scope

The scope including therapeutic of 5-FU concentration from LLOQ to ULOQ value. The analyte signal at ULOQ level should be repeatable and concentration calculated for this solution from the calibration curve equation should be characterised with precision not exceeding 15% CV and accuracy up to 15% of nominal concentration level (Bias). Min. scope: 50% - 150% of therapeutic analyte concentration 0.005 - 0.4 µg/ml; reflecting, adequately, LLOQ and ULOQ.

### D. Recovery

A parameter calculated from comparing the value of a measuring signal obtained after the stage of preparing a biological material sample containing a known quantity of the examined compound with the signal obtained for a liquid solution of the direct standard dosed on the chromatographic unit.

Average recovery of 5-FU calculated for three concentration levels of these compounds is from 80,52% to 84,26%. The parameter value is constant and independent from a blood serum sample and analyte concentration.

### E. Accuracy

It was assessed at a low, average and high concentration level of the substance examined in control samples. The average value from the results does not deviate from the nominal one by more than 15 % (20 % for LLOQ). Result: 0.97 < Bias < 2.53
Direct precision: CV ≤ 2.0%
2.38 < CV < 5.64 %.
Indirect precision: Collective CV ≤ 4,0%
1.97 % < Cv < 7.46%.

### F. Resistance

Resistance was determined by changing of the following parameters:
- changing the mobile phase composition (change of B constituent contribution) by ± 10%,
- change of the column temperature ± 10%,
- change of the mobile phase flow ± 10 %.

Accuracy and precision criteria for analytes quantification in blood serum are complied with despite insignificant changes in conditions of the analytical method -1.30 < Bias < 1.64.

### G. Sample transfer

The test criterion regarding a sample transfer is lack of peaks of the examined substance and IS on the chromatograph of the blank sample dosed after the calibration solution of the highest 5-FU/5-BU concentration. The acceptable maximum sample transfer did not exceed 20% of the concentration value indicated as LLOQ and 5% of the internal standard concentration.

The signal for 5-FU and IS received in the blank sample dosed after the calibration solution at the highest concentration level is, adequately: 16.4%, 8.79% and 0.25%.

### H. Stability of sample solution

Accuracy and precision of analyte quantification content not exceeding 15% of values determined for freshly prepared solution.
-3.1 < Bias < 3.64
0.57 < CV < 3.27

An expert in the field will know how to perform quantification according to the way and in compliance with the invention, based on information provided in the section of examples. The expert will also know how to interpret validation results or repeat quantifications in order to perform it.

The invention-based set includes reagents necessary to prepare solutions the expert will use to proceed according to the invention. The set also includes necessary performance equipment.

## Claims

1. The way of 5-FU quantification in blood serum, with the internal standard, using HPLC with a diode detector, including standard solutions preparation stage, sample preparation and quantification performance, **characteristic by the fact** that the sample is prepared with zirconium sorbing agent and quantification is made on the HYDRO-RP column at 265 nm wave length.

2. Way of 5-FU quantification according to claim 1 including the stages:
a. preparing standard solutions, i.e.:
- preparing solutions of basic standards - basic solution of 5-FU; Cₚ 5-FU of 9.6 mg/ml concentration in water and basic solutions of 5-bromouracil, C_{p IS} of 4.5 mg/ml concentration in 50 % ACN.
- preparing working solution of 5-fluorouracil - working solution I 5-fluorouracil; Cₚ 5-FU in water of 10 ug/ml concentration (Cr 5-FU_I), working solution II 5-fluorouracil; Cₚ 5-FU in water of 1.0 µg/ml concentration (Cr 5-FU_II), working solution III 5-fluorouracil; Cₚ 5-FU in water of 0.1 µg/ml concentration (Cr 5-FU_III)
- preparing working solution of an internal standard (5-bromouracil) - working solution of IS; C_{r IS} in 20% MeOH of 0.058 µg/ml concentration (C_{r IS}).
d. preparing a sample, i.e.:
- sampling 200 µl blood serum and 6.9 µl of 5-FU internal standard solution of 0.058 µg/ml concentration in 20% MeOH (C_{r IS}),
- adding 300 mg of ammonia sulphate, 100 mg zirconium sorbing agent - Z-Sep and 1 ml of 15 % solution of 2-propanol in ethyl acetate,
- stirring such prepared mix for 10 minutes
- centrifuging at 20 000 x g (gravitation) for 5 minutes
- evaporating 600 ul of the extract in nitrogen stream
- reconstituting such obtained dry residue in 200 ul 20 % of methyl alcohol solution;
e. performing an quantification on HPLC, where:
- mobile phase: MeOH/H₂O of 5/95 % vol/vol content adequately
- dosed volume: 10 µl
- measurement temperature: 25 °C
- mobile phase flow speed: 1.0 ml/min

3. The way of monitoring 5-FU concentration in blood serum by means of a series of quantifications defined in claim 1 or 2.

4. The set for quantification performance according to claim 1, including:
- Synergi Hydro-RP column, Phenomenex, dimensions 150 x 4.6 mm, 4 µm grain, pores diameter 80A,
- pre-column, Phenomenex, AQ C18 4x3.0 mm,
- 100 vials with an insert,
- 100 eppendorfs
- mobile phase: 950 ml water to LC and 50 ml MeOH to LC
- 20% solution of MeOH - 20ml
- 15% solution of isopropanol in ethyl acetate - 100ml
- ammonium sulphate - 300g
- sorbing agent - Z-Sep Supelco - 10g
- internal standard of 5-bromouracil
- 5-FU calibrating device
- analysis performance instruction
